# EUROPÄISCHE PATENTSCHRIFT

(11) **EP 0 793 975 B1**
(45) Veröffentlichungstag und Bekanntmachung des Hinweises auf die Patenterteilung: **06.05.2004**
(21) Anmeldenummer: 97250053.2
(22) Anmeldetag: 04.03.1997
(51) Int. Cl.: A61N 1/365

(54) **Vorrichtung zur Steuerung eines Herzstimulators aufgrund der Blutsauerstoffsättigung**
Device for controlling a pacemaker based on the blood oxygen saturation
Dispositif de commande d'un stimulateur cardiaque basé sur la saturation d'oxygène dans le sang

(30) Priorität: 04.03.1996 DE 19609368
(43) Veröffentlichungstag der Anmeldung: 10.09.1997
(73) Patentinhaber: BIOTRONIK Mess- und Therapiegeräte GmbH & Co Ingenieurbüro Berlin, 12359 Berlin (DE)
(72) Erfinder: Neumann, Andreas, Dipl.-Ing., 10969 Berlin (DE); Hardeman, John, Ing., 6865 CP Doorwerth (NL); Wieringa, Fokko, Ing., 6921 JA Duiven (NL)
(74) Vertreter: Eisenführ, Speiser & Partner

(56) Entgegenhaltungen:
- EP-A- 0 222 681
- EP-A- 0 319 158
- EP-A- 0 443 495
- US-A- 5 487 752
- US-A- 5 540 727

## Beschreibung

Die Erfindung betrifft eine Anordnung der im Oberbegriff des Anspruchs 1 angegebenen Art.

Es ist seit langem üblich, die Programmierung eines extern programmierbaren Herzschrittmachers unter Zuhilfenahme eines - insbesondere auch bei unterschiedlichen physischen Belastungen - extrakorporal aufgenommenen EKG mittels eines Programmiergerätes vorzunehmen, wobei der Arzt die Betriebsparameter des Schrittmachers im Hinblick auf die aus dem EKG gewinnbaren Aussagen optimal einzustellen versucht.

Das EKG reflektiert jedoch nur sehr bedingt und indirekt den Zustand des metabolischen bzw. hämodynamischen Systems des Patienten, so daß eine derart vorgenommene Einstellung vielfach nicht wirklich optimal ist. Der Einstellvorgang ist zudem für den Arzt bedien- und zeitaufwendig und birgt Fehlerquellen in sich.

Es ist bekannt, daß bestimmte physikalische oder chemische Größen - etwa die Blutsauerstoffsättigung oder der pH-Wert des Blutes - den hämodynamischen Zustand eines Menschen recht gut widerspiegeln, und diese werden auch zur Steuerung von Herzschrittmachern herangezogen.

Eine Vorrichtung zur Bestimmung der Blutsauerstoffsättigung, die speziell zur intrakardialen Verwendung ausgebildet ist und deren Meßwerte zur Frequenzsteuerung eines Herzschrittmachers herangezogen werden sollen, ist in DE 31 52 963 Cl beschrieben. Diese Vorrichtung umfaßt eine Meßsonde mit einer rot-emittierenden Leuchtdiode (LED) und einem Fototransistor sowie eine Ansteuer- und Signalwandlerschaltung, in der der empfangenen (Rot-)Lichtintensität proportionale Fotostrom unter Zuhilfenahme eines Referenzsignals in ein die Rot-Transmission des Blutes repräsentierendes elektrisches Signal umgewandelt wird. Dieses ist ein Ausdruck der Blutsauerstoffsättigung und damit des hämodynamischen Zustandes des Patienten und kann für die Schrittmachersteuerung genutzt werden.

Das Referenzsignal wird hier in einer zweiten Messung mit umgekehrter Spannungs-Polarität gewonnen, bei der durch eine hierzu vorgesehene Diode im Meßkreis der spezifische Meßsignalanteil eliminiert wird. Der Vergleich von Meß-und Referenzsignal liefert dann den spezifischen Signalanteil.

Das Meßverfahren nach DE 31 52 983 hat den entscheidenden Nachteil, daß die Transmissions- bzw. Reflexionsmessung bei einer einzelnen Meß-Wellenlänge lediglich eine Relativmessung darstellt, mit der zwar Veränderungen der Blutsauerstoffkonzentration, jedoch nicht deren Absolutwert bestimmt werden können. Die Steuerung eines Schrittmachers mit den bei diesem Verfahren gewonnenen Werten kann daher nur eine Trend-Steuerung sein, eine Einstellung von Ausgangswerten bzw. eine Eichung der Betriebsparameter mußte dabei aufgrund anderer Größen erfolgen.

Die Druchschrift US-A-5487752 offenbart eine Vorrichtung gemäß dem Oberbegriff der Anspruchs 1.

Zur Darstellung der physiologischen Zusammenhänge dienen die Figuren 1 bis 3. Von diesen zeigen:
Figur 1 eine grafische Darstellung des spektralen Absorptionsvermögens von Deoxy-Hämoglobin (Hb) und Oxy-Hämoglobin (HbO₂),
Figur 2 eine grafische Darstellung des Absorptionsvermögens von Blut bei 660 nm und bei 950 nm in Abhängigkeit von der Sauerstoffsättigung,
Figur 3 eine grafische Darstellung zum relativen Absorptionsvermögen von Blut in Abhängigkeit von der Sauerstoffsättigung.

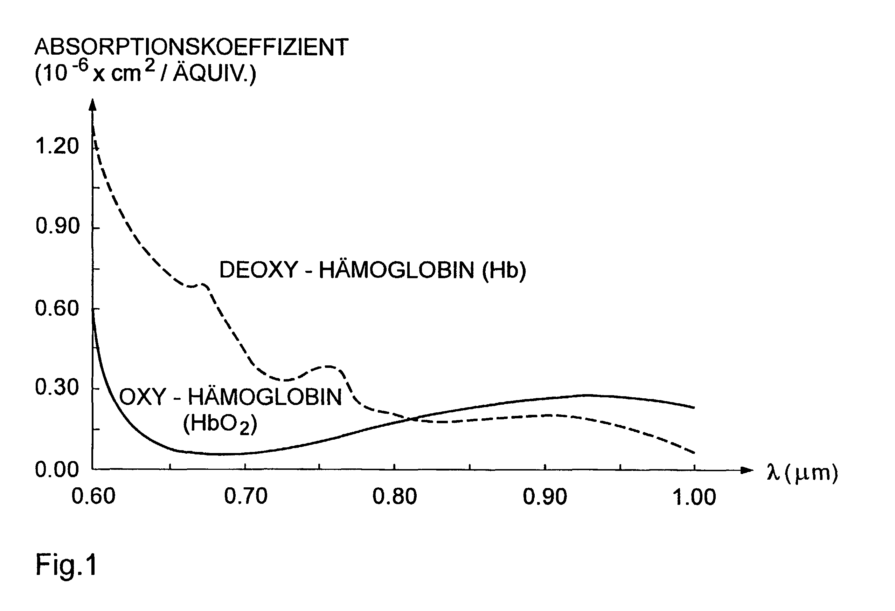

Zur Durchführung einer Absolutmessung der Blutsauerstoffsättigung können die im längerwelligen sichtbaren Bereich und im nahen Infrarot stark unterschiedlichen spektralen Absorptionskurven von Deoxy-Hämoglobin (Hb) und Oxy-Hämoglobin (HbO₂) dienen, die in Fig. 1 gezeigt sind.

In der Figur ist zu erkennen, daß das spektrale Absorptionsvermögen von Hb wie auch von HbO₂ bei 660 bis 670 nm nahezu konstant ist, wobei der Absorptionskoeffizient von Hb hier sehr viel höher ist als derjenige von HbO₂. Bei etwa 810 nm ist das Absorptionsvermögen gleich groß und oberhalb dieser Wellenlänge absorbiert HbO₂ stärker. Eine Vergleichsmessung bei einer Wellenlänge oberhalb von 810 nm gegenüber einer Primär-Messung bei etwa 660 nm ist daher für die Bestimmung des HBO2-Anteils und damit der absoluten Blutsauerstoffsättigung mit hoher Genauigkeit vorteilhaft.

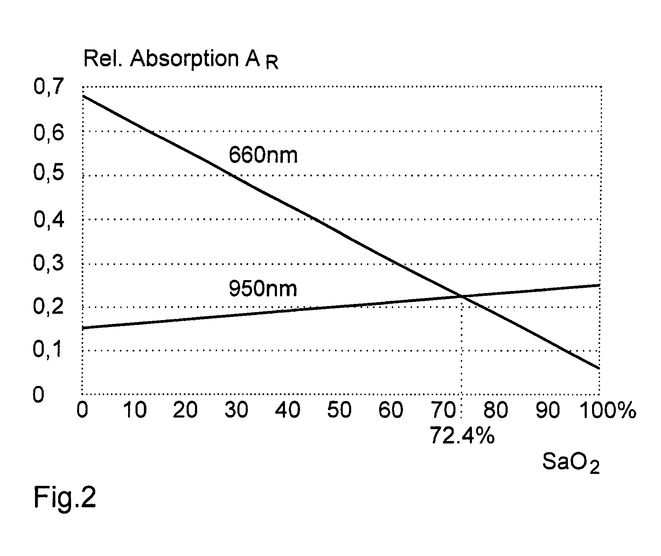

Fig. 2 ist eine grafische Darstellung des relativen Absorptionsvermögens bei 660 nm sowie bei 950 nm in Abhängigkeit von der Blutsauerstoffsättigung SaO₂.

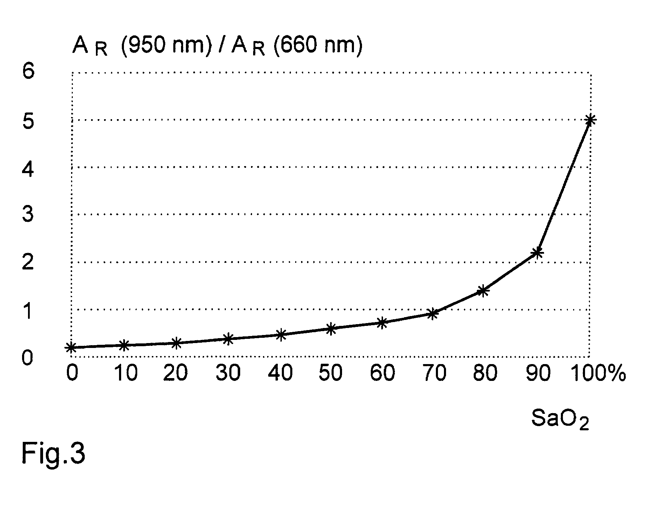

Fig. 3 ist eine vom Informationsgehalt im Prinzip zu Fig. 3 äquivalente Darstellung, bei der der Quotient aus Infrarot(IR)- und Rot(R)-Absorption bei den genannten Wellenlängen über SaO₂ aufgetragen ist. Die Kreuze bezeichnen Werte des Quotienten in 10%-Schritten, und diese wurden in der Figur durch Geraden verbunden; die eigentliche Funktionskurve ist eine Hyperbel.

Aus EP 0 257 954 Bl ist eine Weiterentwicklung des Meßverfahrens und der Meßanordnung nach DE 31 52 993 bekannt, die offensichtlich die vorstehend beschriebenen Zusammenhänge ausnutzt und bei der nunmehr ein Zwei-Wellenlängen-Reflexionsoximeter in einer Schrittmacherleitung integriert ist, dessen Ausganggsignale die tatsächliche Blutsauerstoffsättigung reflektieren und zu einer verbesserten Steuerung eines Bedarfsschrittmachers dienen.

Die Meßanordnung umfaßt einen Fotodetektor, der gleichermaßen zum Nachweis der Transmission im roten und im infraroten Spektralbereich genutzt wird. Dies erfordert jedoch einen hohen Aufwand bei der Ansteuerung der Meßanordnung und das Vorsehen von Meßsignalspeichern.

Aus US 4 712 179 sind ein Verfahren und eine Vorrichtung zur Korrektur der über intrakardiale Meßanordnungen im Zusammenwirken mit einem implantierten Schrittmacher gewonnenen Meßwerte unter Zuhilfenahme eines externen Programmierund Auswertungsgerätes bekannt. Hiermit erfolgt eine Befreiung intrakardial aufgenommener Meßwerte von Verfälschungen, die aus ungenauer Dimensionierung von Dickschicht-Widerständen bei der Herstellung der Schrittmacherschaltkreise resultieren, indem für jeden hergestellten Schrittmacher exemplarspezifische Korrekturwerte ermittelt, in einem ROM-Speicher im Schrittmacher gespeichert, bei der Übertragung der Meßwerte an das Programmier- und Auswertungsgerät mit übertragen und in diesem zur Berechnung korrigierter Meßwerte herangezogen werden.

Der Erfindung liegt die Aufgabe zugrunde, eine Anordnung der eingangs genannten Gattung anzugeben, mit der eine Einstellung bzw. Eichung eines Herzstimulators aufgrund von über eine externe Meßvorrichtung gewonnenen, den metabolischen Zustand des Patienten zuverlässig reflektierenden Meßwerten möglich ist und die konstruktiv einfach und variabel ausgebildet und vielfältig eingesetzt werden kann.

Diese Aufgabe wird durch eine Anordnung mit den Merkmalen des Anspruchs 1 gelöst.

Die Erfindung schließt den Gedanken ein, Betriebsparameter eines Herzstimulators - Herzschrittmachers oder Defibrillators bzw. Kardioverters - aufgrund von mit hinreichender Genauigkeit extern gewonnenen Absolutwerten einer für den metabolischen Zustand des Patienten repräsentativen, nicht-elektrischen Größe im Zuge eines Programmiervorganges vorzugeben bzw. zu eichen und ein dazu geeignetes automatisches System anzugeben. Diese Parameter können insbesondere die Stimulationsrate und/oder die AV-Verzögerungszeit sein.

Die Erfindung schließt weiter das Vorsehen einer Meßvorrichtung ein, in der mit einem sichtbares Licht aussendenden und einem das sichtbare Licht empfangenden Bauelement ein erster Meßkanal sowie mit einem Infrarotstrahlung aussendenden und einem diese empfangenden Bauelement ein zweiter, vom ersten getrennter, Meßkanal gebildet ist. Sie schließt weiter den Gedanken ein, die Steuer- und Auswertungseinrichtung des Meßgerätes so auszubilden, daß beide Meßkanäle gleichzeitig betrieben und die darin gewonnenen Meßsignale im wesentlichen gleichzeitig verarbeitet werden können. Damit kann auf Umschaltungen und Signal-Zwischenspeicherungen grundsätzlich verzichtet werden, womit Fehlerquellen eliminiert werden und ein schneller und störungsarmer Betrieb gewährleistet wird.

Die Steuer- und Auswertungseinrichtung des Meßgerätes kann damit so ausgebildet werden, daß die Meßsignale zeitnah (on-line) verarbeitet werden können. Dies ermöglicht die schnelle Aufnahme von Meßkurven zur Änderung der Blutsauerstoffsättigung - oder einer anderen repräsentativen Meßgröße - in Reaktion auf die Variation von Betriebsparametern des Herzstimulators und damit eine zügige Optimierung der entsprechenden Parameter anhand der Meßwerte.

Die Genauigkeit der Meßwerte wird - für den Fall der Meßgröße Blutsauerstoffsättigung - dadurch weiter erhöht, daß die aussendenden und/oder die empfangenden Bauelemente so ausgebildet und/oder derart mit Filtermitteln versehen sind, daß das Spektrum des empfangenen Lichts und dasjenige der empfangenen Infrarotstrahlung im wesentlichen keinen Überlappungsbereich aufweisen. Je ausgeprägter die Trennung der beiden Meßbereiche ist, desto größer kann angesichts der in Fig. 1 dargestellten Absorptionskurven die Meßgenauigkeit sein.

Die Erfindung schließt dazu weiter den Gedanken ein, einen Eingang des Programmiergerätes mit dem Ausgang einer Steuer- und Auswertungseinrichtung für die nicht-elektrische Größe - etwa die Blutsauerstoffsättigung - zu verbinden und das Programmiergerät mit Mitteln zur selbsttätigen Durchführung und Auswertung einer Meßreihe für die nicht-elektrische Größe in Abhängigkeit von verschiedenen, über die Telemetrieeinrichtung am Herzstimulator eingestellten Werten des Betriebsparameters und Festlegung eines optimierten Betriebsparameters entsprechend dem Auswertungsergebnis der Meßreihe zu versehen.

Diese Mittel umfassen in zweckmäßigen Ausgestaltungen einen, insbesondere als Matrixspeicher ausgeführten, Hilfsspeicher zur Speicherung von Meßwerten der nicht-elektrischen Größe in Zuordnung zu Betriebsparameterwerten, nach deren Einstellung sie aufgenommen wurden, eine Vergleichereinheit zur Auswertung der Meßwerte der nicht-elektrischen Größe mit Hilfe von in einem, mit einem Steuereingang der Vergleichereinheit verbundenen, Kriterien-Festwertspeicher zur Speicherung mindestens eines Vergleichskriteriums für die Auswertung und eine Zeitablaufsteuerung und einen Zähler zur zeitlichen Steuerung des Ablaufs einer Meßreihe derart, daß in einem vorbestimmten Zeittakt in vorbestimmter Folge verschiedene Betriebsparameterwerte am Schrittmacher eingestellt und zusammen mit dem jeweils erhaltenen Meßwert der nicht-elektrischen Größe ausgegeben bzw. gespeichert werden.

Die Meßanordnung ist insbesondere so ausgebildet, daß die Ausgangssignale direkt on-line verarbeitet werden können.

Als nicht-elektrische Meßgröße ist insbesondere die Blutsauerstoffsättigung geeignet, wobei ein Meßverfahren anzuwenden ist, das die Bestimmung von deren Absolutwert erlaubt.

Eine hierfür geeignete Meßanordnung umfaßt mindestens je ein sichtbares Licht sowie Infrarotstrahlung aussendendes Bauelement sowie ein das sichtbare Licht und die Infrarotstrahlung nach Durchgang durch einen durchbluteten Körperbereich empfangendes Bauelement, wobei das das sichtbare Licht aussendende und das dieses empfangende Bauelement einen ersten Meßkanal sowie das die Infrarotstrahlung aussendende und das diese empfangende Bauelement einen zweiten, vom ersten getrennten, Meßkanal bilden und der erste und der zweite Meßkanal gleichzeitig betrieben und die in beiden Meßkanälen erhaltenen Meßsignale im wesentlichen gleichzeitig verarbeitet werden.

Von Bedeutung für eine gute Kanaltrennung und damit für die Genauigkeit der Absolutmessung ist, daß die aussendenden und/oder die empfangenden Bauelemente so ausgebildet und/oder derart mit Filtermitteln versehen sind, daß die Spektren der jeweisl empfangenen Strahlung im wesentlichen keinen Überlappungsbereich aufweisen.

Das sichtbare Licht hat in Anbetracht der in Fig. 1 gezeigten Kurven vorteilhafterweise ein Intensitätsmaximum bei einer Wellenlänge von etwa 660 nm und die Infrarotstrahlung ein Intensitätsmaximum bei einer Wellenlänge von etwa 950 nm.

Eine kostengünstige und kompakte und damit vielseitige Einsatzmöglichkeiten erschließende Realisierung der optoelektronischen Bauelemente besteht darin, daß die aussendenden Bauelemente mindestens je eine rot- und eine infrarotemittierende LED oder Laserdiode und die empfangenden Bauelemente ein rot- und ein infrarotempfindlicher Halbleiter-Fotodetektor sind. In einer speziellen, lichtstarken und den störenden Einfluß lokaler Inhomogenitäten des Gewebes bzw. des Blutstromes weitgehend eliminierenden Ausführung sind je drei rot- und infrarotemittierende LEDs bzw. Laserdioden vorgesehen.

Um die oben erwähnte Meßbereichstrennung sowie eine Ausschaltung störender kurzwelliger Strahlungsanteile bei leicht verfügbaren und kostengünstigen Standard-Bauelementen zu realisieren, kann dem das sichtbare Licht empfangenden Bauelement ein optisches Bandfilter und dem die Infrarotstrahlung empfangenden Bauelement ein Filter für kurzwellige Strahlungsanteile zugeordnet sein.

In der Steuer- und Auswertungseinrichtung können in vorteilhafter Weise Mittel zum Erzeugen und Anlegen einer Wechselspannung an die aussendenden Bauelemente zur Modulation der emittierten Strahlung sowie mindestens eine Verarbeitungsstufe zur Auswertung des modulierten Anteils der empfangenen Strahlung vorgesehen sein. Damit ist zum einen die Bestimmung harmonischer Verzerrungen der empfangenen Strahlung und zum anderen die Bestimmung des Transmissionsgrades der empfangenen Strahlung möglich. Dies ermöglicht es, einen Betrieb der Anordnung im nichtlinearen Bereich der Bauelemente (etwa bei Patienten mit sehr "durchscheinender" Haut), der zu einer Verfälschung der Meßwerte führen würde, zu erkennen und durch entsprechende Nachjustierung abzustellen.

Die Verarbeitungsstufen können speziell jeweils ein Bandfilter und eine Gleichrichterstufe, deren Ausgangssignale - je nach Wahl des Filter-Durchlaßbereiches - ein Maß für die harmonischen Verzerrungen oder den Transmissionsgrad des durchstrahlten Gewebes sind, und eine Vergleichereinheit aufweisen, in der das entsprechend verarbeitete Empfangs-Pilotsignal mit dem Sende-Pilotsignal verglichen wird.

Die Ergebnisse können dem Bediener angezeigt werden, woraufhin dieser eine manuelle Nachjustierung vornehmen kann. Vorteilhafter ist aber das Vorsehen einer automatischen Justierung des Sendestromes nach Maßgabe der Vergleichsergebnisse auf einen Wert, bei dem die Detektoren in einem annähernd linearen Bereich arbeiten. Die ermittelten Werte bzw. die Justierstellung können patientenbezogen gespeichert und für spätere Messungen verwendet werden.

Im eigentlichen Meß-Signalweg (des unmodulierten Signalanteils) zur Bestimmung der Blutsauerstoffsättigung ist in einer einfachen Ausführung mit kostengünstigen Operationsverstärkern zunächst ein Tiefpaßfilter (etwa mit f_{go} = 40 Hz) mit einem ersten Operationsverstärker und ein Hochpaßfilter (etwa mit f_{gu} = 0,1 Hz - zur Eliminierung langsamer Nullpunktdrift) mit einem zweitem Operationsverstärker vorgesehen. Die Filter arbeiten vorzugsweise als Bessel-Filter mit minimaler Phasenverzerrung, und die Schaltung kann die Eigenschaften eines NIC-Konverters aufweisen.

Die Meßvorrichtung kann im äußeren Aufbau für die Messung an der Körperoberfläche ausgebildet sein, indem die aussendenden und die empfangenden Bauelemente mit einem Abstandsbereich voneinander in einem (auch flexiblen) Gehäuse angeordnet sind, das zur Aufnahme eines Körperteils, insbesondere eines Ohrläppchens oder Fingers, eines Patienten im Abstandsbereich ausgebildet ist.

Es ist aber auch möglich, die aussendenden und die empfangenden Bauelemente mit einem Abstandsbereich voneinander in einer miniaturisierten Baugruppe anzuordnen, die zur intrakorporalen Messung, insbesondere im Herzen oder in einem größeren Gefäß, ausgebildet ist.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

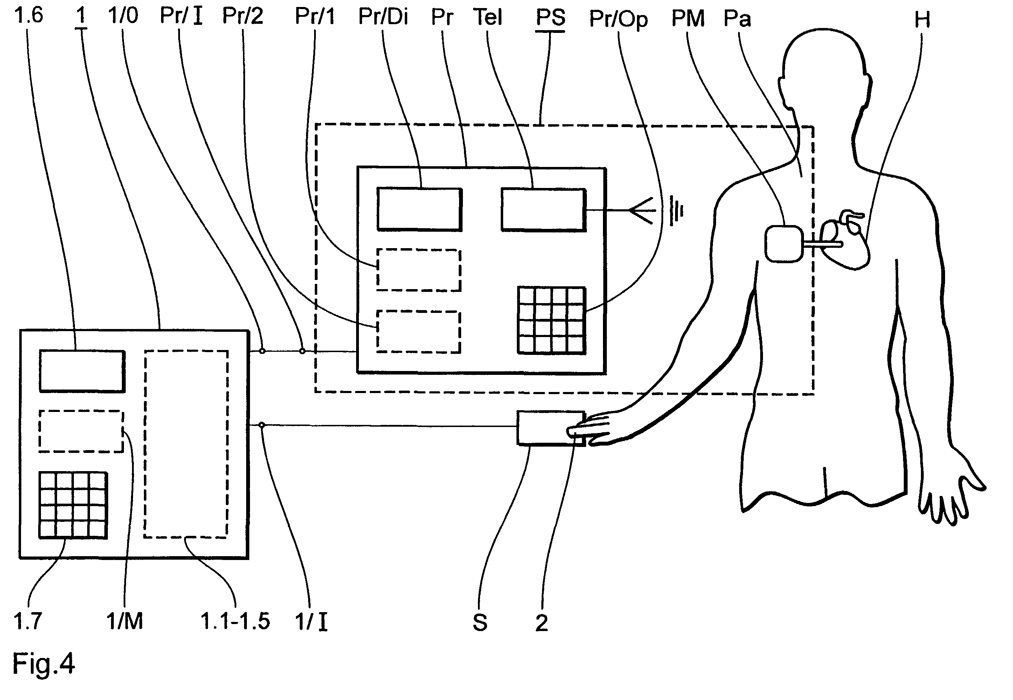

Figur 4 eine schematische Darstellung der erfindungsgemäßen Anordnung in einer Ausführungsform,

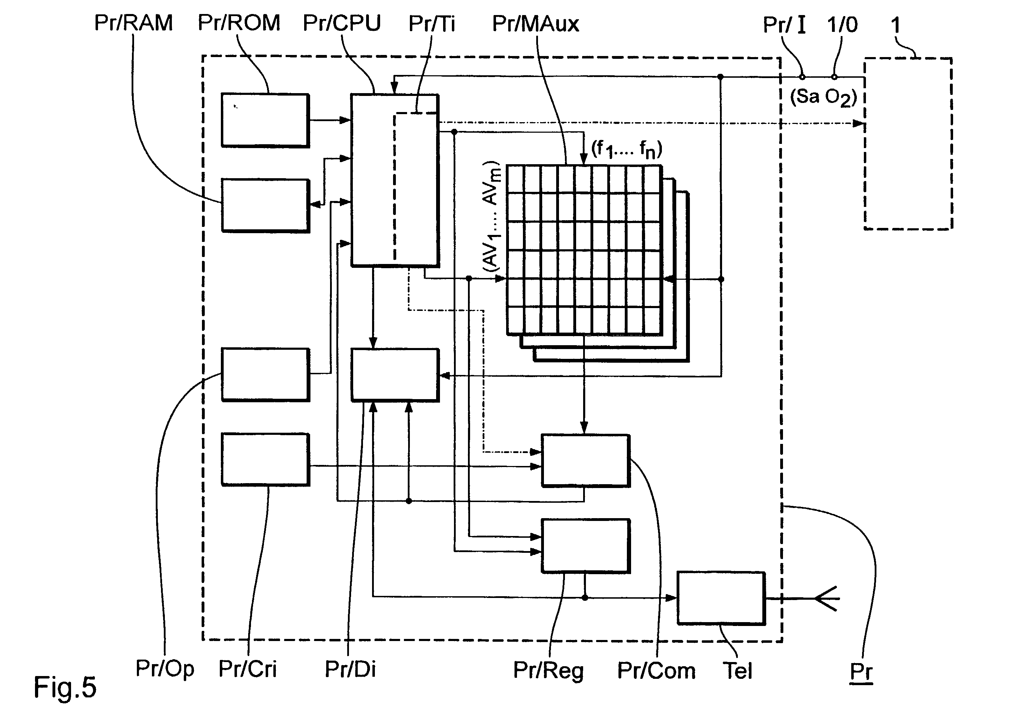

Figur 5 ein vereinfachtes Blockschaltbild zur Verdeutlichung des Zusammenwirkens von Komponenten des Herzschrittmachers und des Programmiergerätes in einer gegenüber Fig. 4 modifizierten Konfiguration,

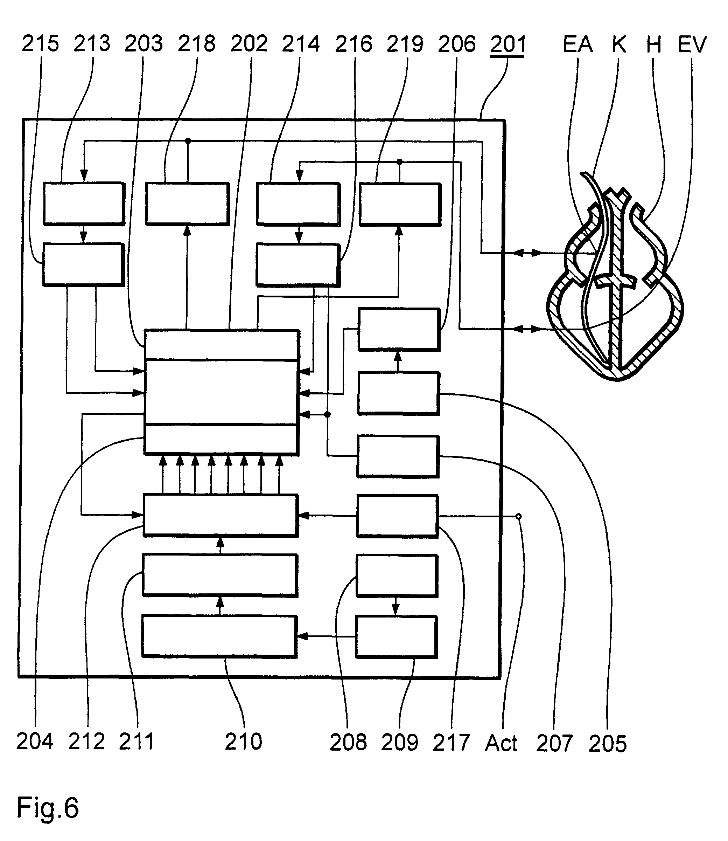

Figur 6 ein vereinfachtes Blockschaltbild eines AVsequentiellen Zweikammerschrittmachers, der mit einer Anordnung nach einer Ausführungsform der Erfindung eingestellt bzw. geeicht werden kann,

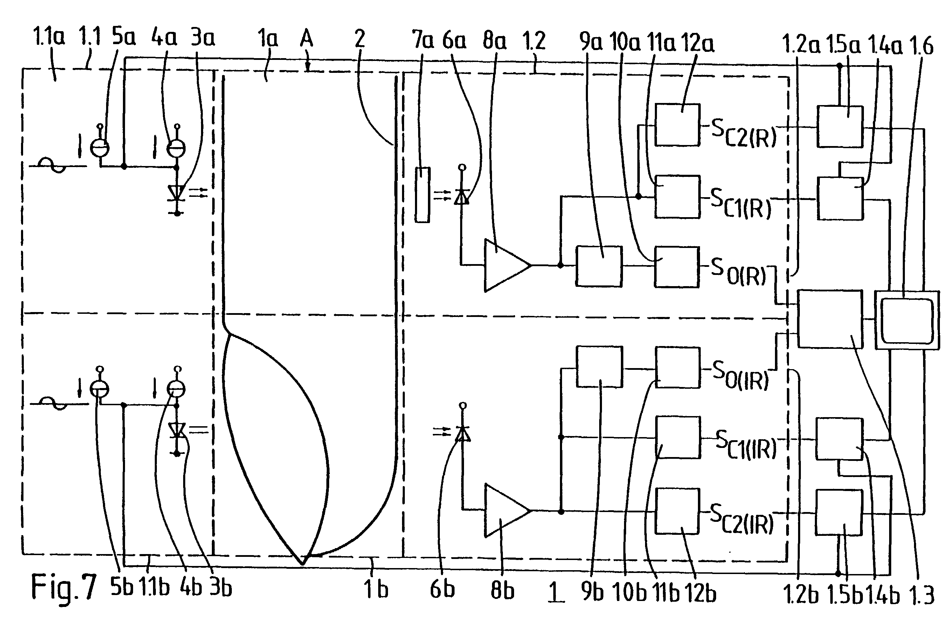

Figur 7 ein vereinfachtes Blockschaltbild einer Vorrichtung zur Bestimmung der Blutsauerstoffsättigung als Teil einer Anordnung gemäß einer Ausführungsform,

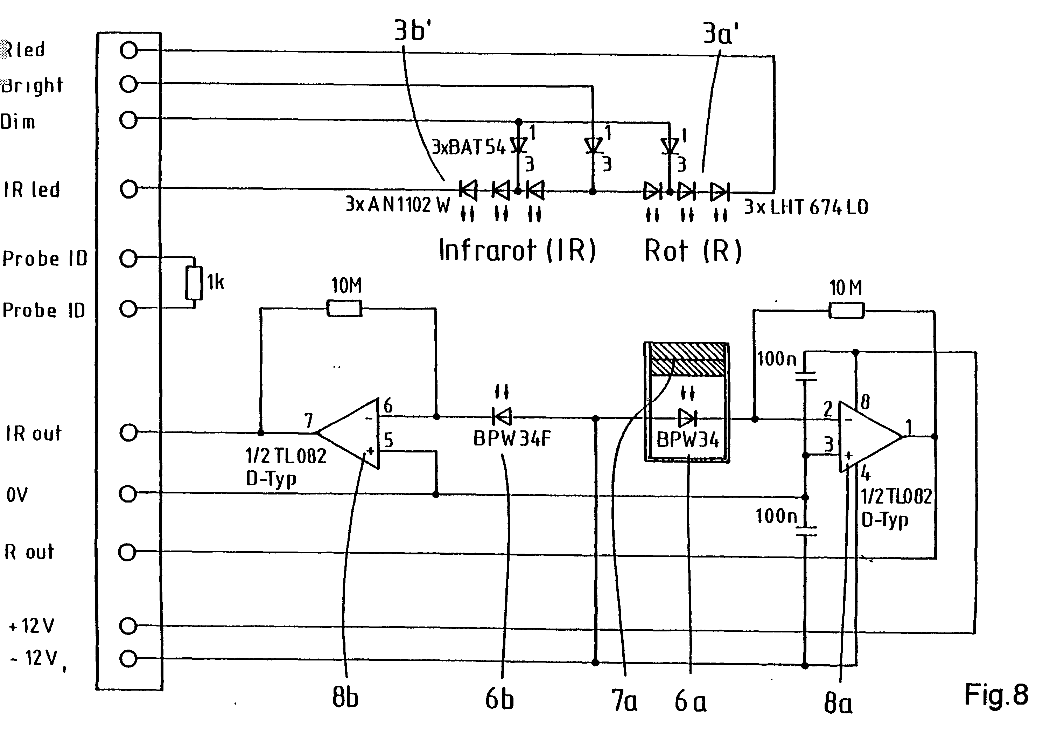

Figur 8 eine Detaildarstellung der Vorrichtung nach Fig. 7,

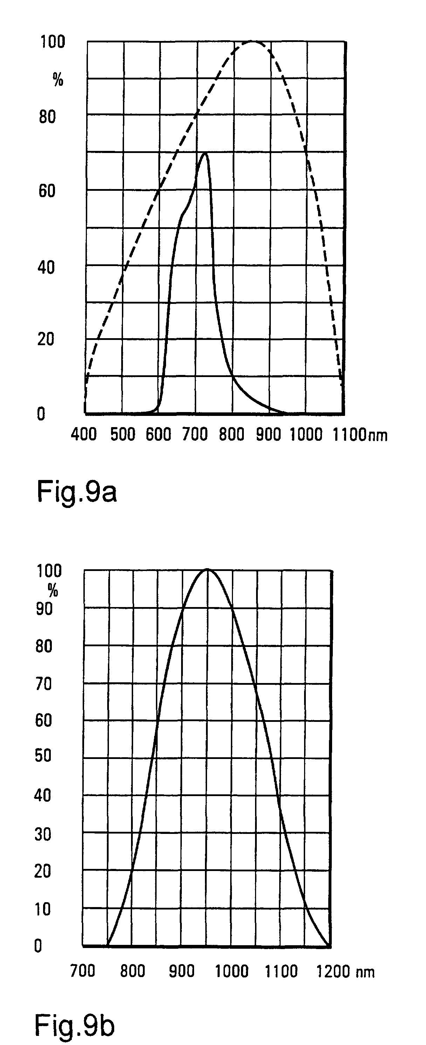

Figur 9a und 9b Darstellungen der Empfindlichkeitskurven der Licht- bzw. IR-Empfangs-Bauelemente mit vorgeschaltetem bzw. integriertem optischem Filter bei einer speziellen Ausbildung der in Fig. 7 gezeigten Vorrichtung,

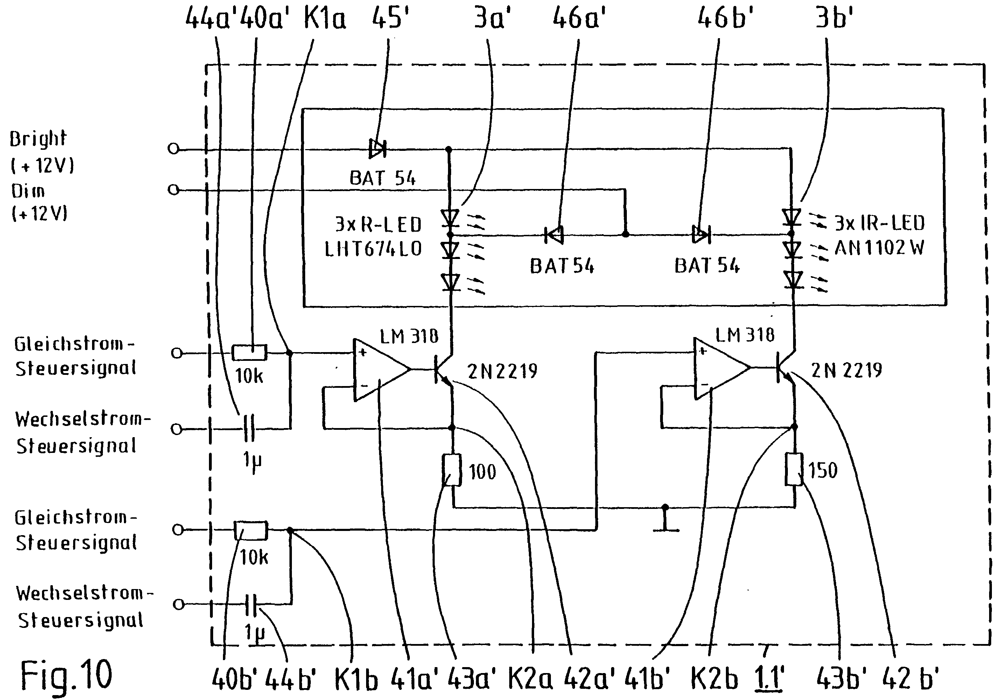

Figur 10 eine Detaildarstellung der LED-Ansteuerung bei einer modifizierten Vorrichtung nach Fig. 7 und

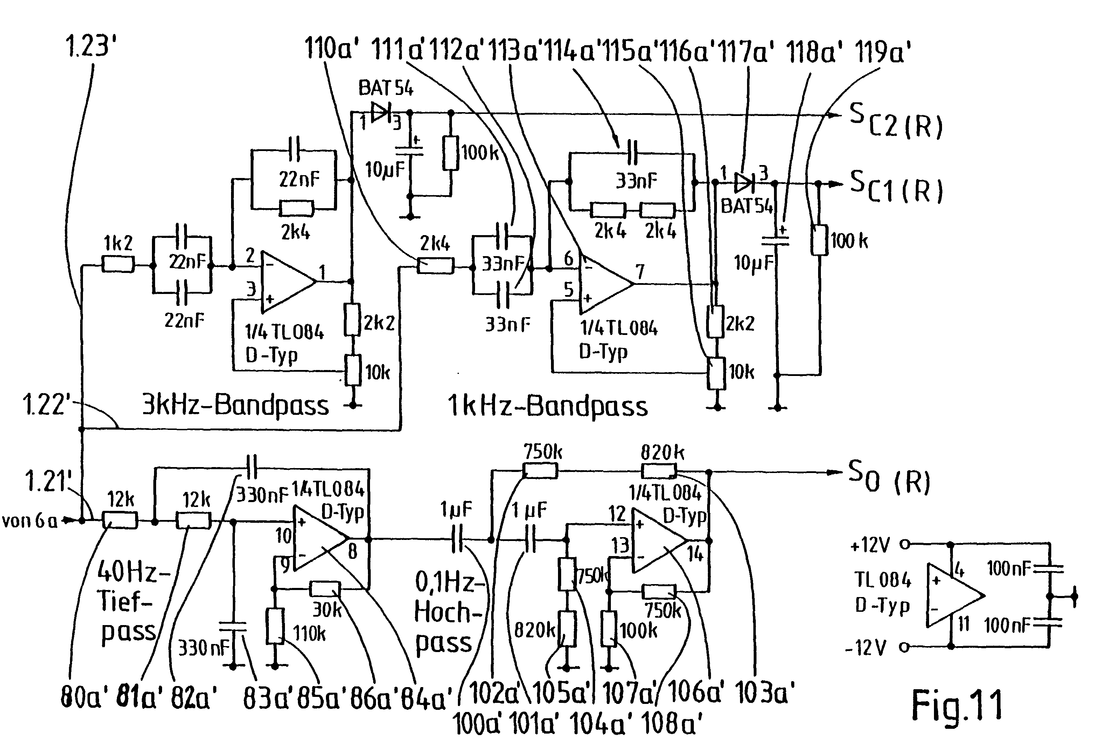

Figur 11 eine Detaildarstellung der Auswertungsschaltung bei einer modifizierten Vorrichtung nach Fig. 7.

Figur 4 ist eine schematische Darstellung einer Anordnung zur Steuerung bzw. Programmierung eines Herzschrittmachersystems PS aus einem Programmiergerät Pr und einem in einen Patienten Pa implantierten Schrittmacher PM. Das Programmiergerät weist eine Bedieneinheit Pr/Op und eine Anzeigeeinheit Pr/Di auf, und innerhalb des Gerätes sind zwei Einstellfunktionen durch Blöcke Pr/1 bzw. Pr/2 symbolisiert. Schrittmacher PM und Programmiergerät Pr stehen über eine Telemetrieeinheit Tel, über die am Programmiergerät vorgenommene Betriebsparametereinstellungen an den Schrittmacher übertragen werden, miteinander in Verbindung.

Als Gerät zur Messung einer den metabolischen bzw. hämodynamischen Zustand des Patienten reflektierenden Größe ist ein Pulsoximeter 1 zur Erfassung der Blutsauerstoffsättigung vorgesehen. Zu diesem gehört eine Meßsonde S, die einen Finger 2 des Patienten Pa aufnimmt. Die Meßsonde S ist an einen Eingang 1/I des Pulsoximeters 1 angeschlossen, und dieses ist über einen Ausgang 1/O mit einem Eingang Pr/I der Programmiereinheit Pr des Schrittmachersystems PS verbunden.

Das Pulsoximeter 1 ist in den Figuren 7 bis 11 genauer gezeigt und im zugehörigen Teil der Bechreibung ausführlich beschrieben. In Fig.4 ist von dessen Aufbau eine Anzeigeeinheit 1.6 und eine Bedieneinheit 1.7 einzeln gezeigt; die in Fig. 7 darstellten Baugruppen 1.1 bis 1.5 sind in Fig. 4 zuammenfassend als ein (gestrichelt gezeichneter) Block symbolisiert. Weiterhin ist im Pulsoximeter 1 ein Meßwertund Einstellungsspeicher 1/M vorgesehen.

Das Zusammenwirken des Pulsoximeters 1 mit dem Schrittmachersystem PS bei einer Routineuntersuchung zur Überprüfung der Funktionstüchtigkeit und der Programmparameter des Schrittmachers ist (im Sinne eines Beispiels) wie folgt:

Im Laufe der Überprüfung der Einstellungen des Schrittmachersystems wird über das Bedienfeld Pr/Op die erste extern, d.h. über die Programmiereinheit Pr, zu realisierende Einstellfunktion Pr/1 aufgerufen, die einen Bezug zum hämodynamischen Zustand des Patienten Pa hat, etwa die Einstellung der Stimulationsrate. Mit der bis zur Untersuchung gültigen Einstellung sowie weiteren im System vorgesehenen Grundeinstellungen wird eine Meßreihe gebildet. In jedem Schritt der Meßreihe wird ein Einstellwert über die Telemetrieeinheit Tel an den Schrittmacher PM übertragen und von diesem über eine am Programmiergerät Pr vorgegebene Zeitspanne das Herz H des Patienten Pa mit diesem Wert stimuliert.

Nach Verstreichen einer vorbestimmten Zeitspanne (die für das Pulsoximeter etwa durch Übertragung eines Auslösesignals durch das Programmiergerät signalisiert wird) wird durch das Pulsoximeter 1 über die Meßsonde S eine Messung der Blutsauerstoffsättigung vorgenommen, und der für den eingestellten Stimulationsratenwert erhaltene Blutsauerstoffsättigungswert wird in Zuordnung zum Ratenwert im Programmiergerät gespeichert, wobei die Nummer der Messung in der Meßreihe oder direkt der Raten- bzw. Frequenzwert als Adresse dienen kann.

Danach wird der nächste Wert der Stimulationsrate aufgerufen und das beschriebene Vorgehen für diesen wiederholt.

Gleichzeitig wird bei den Messungen im Speicher 1/M auch der während der Messung automatisch eingestellte Sendestrom für die (in Figur 4 nicht gezeigten) LEDs in der Meßsonde S unter dem Patientennamen abgelegt, so daß bei späteren analogen Untersuchungen bei demselben Patienten sogleich von den korrekten Einstellungen der Sensoranordnung ausgegangen werden kann.

Nach Abschluß der Meßreihe - bei gleichbleibender Belastung des Patienten - wird (auf weiter unten genauer beschriebene Weise) im Programmiergerät automatisch der höchste Wert der Blutsauerstoffsättigung ermittelt, im internen Speicher 1/M des Pulsoximeters 1 unter dem Patientennamen abgelegt und der zugehörige Ratenwert über das Programmiergerät Pr als neuer, endgültiger Einstellwert für die mit der Meßreihe untersuchte Belastungsstufe des Patienten an den Schrittmacher PM übermittelt.

Auf völlig analoge Weise können, wenn im Rahmen der Untersuchung eine Überprüfung bzw. Eichung der Belastungs-Raten-Kennlinie eines frequenzadaptiven Schrittmachers vorgenommen werden soll, weitere Meßreihen bei veränderter Belastung des Patienten durchgeführt und ausgewertet und der optimierte Ratenwert für jeden Belastungszustand an den Schrittmacher als neue permanente Einstellung übermittelt werden. Im Schrittmacher werden die einzelnen Ratenwerte zusammen mit einer Kennung für den Aktivitäts-bzw. Belastungszustand, dem sie zuzuordnen sind, abgespei-chert und stehen dann für den Normalbetrieb abrufbar zur Verfügung.

Ebenfalls grundsätzlich analog kann in einer weiteren Meßreihe oder Serie von Meßreihen die zweite Einstellung Pr/2, etwa die AV-Verzögerung eines Zweikammerschrittmachers PM (der unten genauer beschrieben wird) bei einem oder mehreren vorgegebenen Ratenwert(en), optimiert werden.

Nach der erfolgten Neueinstellung erfolgt die Steuerung des Schrittmachers dann derart, daß über einen ihm zugeordneten, implantierten Aktivitäts- oder Belastungssensor ein Signal abgegeben wird, aus dem die oben erwähnte Kennung für den Belastungszustand gewonnen wird, und daß der zugehörige gespeicherte Raten- und AV-Delay-Wert eingestellt wird. (Auch dies wird weiter unten genauer erläutert.)

Figur 5 ist ein stark vereinfachtes Blockschaltbild zur Verdeutlichung von Aufbau und Funktion des Programmiergerätes Pr in einer (funktionell leicht modifizierten) Konfiguration nach Fig. 4. Die Anordnung unterscheidet sich von der oben beschriebenen darin, daß sie eine Optimierung von Rate und AV-Verzögerungszeit mit einem veränderten Ablauf erlaubt.

Herzstück des Programmiergerätes Pr ist eine Mikroprozessoreinheit Pr/CPU, der in an sich bekannter Weise ein Programmspeicher Pr/ROM und ein Datenspeicher Pr/RAM sowie - wie bereits in Fig. 4 gezeigt - eine Anzeigeeinheit Pr/Di und ein Bedienfeld Pr/Op zugeordnet sind. Von spezieller Bedeutung für die Durchführung der Überprüfung/Neueinstellung der (hier beispielhaft gewählten) Schrittmacher-Betriebsparameter AV-Verzögerung und Stimulationsrate ist ein in mehreren Ebenen jeweils matrixartig organisierter Hilfsspeicher Pr/MAux. Dessen Spaltenadressen sind die einzelnen im Datenspeicher Pr/RAM unter Spezifizierung für den implantierten Schrittmacher verfügbaren Stimulationsfrequenzwerte f₁ ... fₙ und dessen Zeilenadressen die jeweils verfügbaren AV-Verzögerungswerte AV₁ ... AVₙ. Die Speicher-Ebenen stehen hier für die einzelnen Messungen einer kombinierten Raten-/AV-Delay-Meßreihe.

Als Speicherwert wird in ein jeweils adressiertes Speicherfeld der für eine bestimmte Kombination von Frequenz-und AV-Delay-Wert gemessene Blutsauerstoff-Sättigungswert SaO2 eingespeichert. (Für eine Gesamtdarstellung sowie Einzelheiten der Organisation und Handhabung derartiger Speicher für Betriebsparameter-Kennfelder wird hier auf EP-B-0 222 681 verwiesen.)

Weiterhin weist das Programmiergerät Pr als im gegebenen Zusammenhang funktionsentscheidende Baugruppe eine eingangsseitig mit dem Datenausgang des Hilfsspeichers Pr/MAux und ausgangsseitig mit einem Betriebsparameter-Register Pr/Reg verbundene Vergleichereinheit Pr/Com zur Auswertung der Meßwerte der Blutsauerstoffsättigung auf. Außerdem umfaßt sie einen mit einem Steuereingang der Vergleichereinheit Pr/Com verbundenen Kriterien-Festwertspeicher (ROM) Pr/MCri zur Speicherung eines oder mehrerer Vergleichskriterien oder -werte als Grundlage für die Auswertung der Blutsauerstoffsättigung sowie eine - in der Figur als Teil der Pr/CPU gezeigte - Zeitablaufsteuerung Pr/Ti für die Ausführung der Messungen und der aich anschließenden Vergleichsprozedur.

Zur Neueinstellung bzw. Eichung des Schrittmachers wird grundsätzlich das oben unter Bezugnahme auf Fig. 4 beschriebene Vorgehen praktiziert; Stimulationsrate und AV-Verzögerung werden hier aber nicht nacheinander in zwei getrennten Meßreihenserien optimiert, sondern (quasi "zweidimensional") in einer Meßreihenserie.

Über das Bedienfeld Pr/Op des Programmiergerätes wird zunächst eine Betriebsart und eine zu optimierende Betriebsparameter-Korrelation - in diesem Falle die Korrelation f/AV - vorgewählt und aus dem Programmspeicher Pr/ROM gelesen. Dabei werden zugleich automatisch die Sätze f₁...fₙ und AV₁...AVₘ verfügbarer Betriebsparameter aus dem Datenspeicher Pr/RAM ausgelesen und die aktuelle Organisation des Hilfsspeichers Pr/MAux vorgegeben.

Dann wird auf einer (in den Figuren nicht gezeigten) ergometrischen Apparatur eine erste Belastungsstufe des Patienten (beispielsweise der Ruhezustand) eingestellt, und für diesen Zustand werden - z.B. ausgehend von einem bei einer früheren Untersuchung für diesen Belastungszustand als optimal ermittelten Parameterpaar (fᵢ, AVᵢ) und unter Einschluß der diesem nächst (und ggfs. übernächst) benachbarten Paare oder auch einfach unter "Abrastern" aller hinreichend sinnvollen n Parameterpaare - nacheinander Paare (fᵢ, AVᵢ) mit i= 1, 2, ..., n vorgewählt. Das vorgewählte Parameterpaar wird in das Betriebsparameter-Register Pr/Reg übernommen und über die Telemtrieeienhiet Tel an den Schrittmacher PM übermittelt. Dort wird der Impulserzeuger jeweils für eine bestimmte, durch die Zeitsteuereinheit Pr/Ti des Programmiergerätes vorgegebene Zeitdauer (deren Ablauf über die Telemetrieeinheit einfach durch Übermittlung eines neuen Parameterpaares signalisiert wird) eingestellt. Während die Reizimpulse mit diesen Parametern an das Herz des Patienten übertragen werden, wird nach einer kurzen, ebenfalls über die Zeitsteuereinheit Pr/Ti vorgegebenen, Wartezeit zur Einstellung eines hämodynamischen Gleichgewichts auf die weiter unten beschriebene Weise die Blutsauerstoffsättigung gemessen und der Meßwert unter der Adresse (fᵢ, AVᵢ) in den Hilfsspeicher Pr/MAux übernommen sowie gleichzeitig (zusammen mit den aktuellen Parametern) auf der Anzeigeeinrichtung Pr/Di für den Arzt dargestellt.

Sobald der Meßwert vorliegt, wird zum nächsten Paar (fᵢ₊₁, AVᵢ₊₁) weitergegangen, indem die Steuereinheit Pr/CPU, nachdem sie das Vorliegen des Meßwertes festgestellt hat, die nächste Adresse in Pr/MAux aufruft.

Sind die Messungen für die erste Belastungsstufe abgeschlossen, d.h. alle relevanten Adressen der ersten Ebene von Pr/MAux abgearbeitet, werden die Speicherwerte unter Ablaufsteuerung durch die CPU sukzessive jeweils paarweise in die Vergleichereinheit Pr/Com übernommen und dort nach einem aus dem Kriterienspeicher Pr/Cri übernommenen Vergleichskriterium (hier einfach SaO₂ = max.) verglichen, bis der höchste SaO₂-Wert gefunden ist. Die Ergebnisse des Vergleiches können auf der Anzeigeeinheit Pr/Di dargestellt werden.

Parallel zur Übernahme eines SaO₂-Wertes in die Vergleichereinheit wird jeweils die zugehörige Adresse in das Betriebsparameter-Register Pr/Reg geladen, so daß sich am Ende der Vergleichsprozedur diejenige Adresse in diesem Register befindet, die zum höchsten SaO₂-Wert gehört und die optimale Betriebsparameter-Kombination f/AV für den zugrundegelegten Belastungszustand darstellt. Diese wird auf der Anzeigeeinhiet Pr/Di dargestellt und über die Telemetrieeinheit an den Schrittmacher übertragen, wo sie (wie weiter unten genauer erläutert) gespeichert wird.

Diese Messungen werden für eine Mehrzahl vorgegebener Belastungsstufen jeweils auf analoge Weise ausgeführt und ausgewertet. (Die Auswertung kann aber alternativ auch nach Abschluß aller Messungen vorgenommen werden.) Im Ergebnis liegt im Schrittmacher für die gewählte Betriebsart ein hämodynamisch optimierter Satz von Parameter-Paaren (fₖ, AVₖ) vor, aufgrund dessen die belastungsabhängige Steuerung des Schrittmachers in der Zukunft erfolgen kann.

Figur 6 zeigt ein vereinfachtes Blockschaltbild eines AVsequentiellen Zweikammerschrittmachers 201, der mit einer Anordnung nach einer Ausführungsform der Erfindung eingestellt bzw. geeicht werden kann,

Dieser hat einen weitgehend bekannten Aufbau, der insoweit daher nur knapp skizziert wird: Kernstück ist eine Steuereinheit 202, der eine Schaltung 203 zum "Run-away"Schutz und ein Programmdecoder 204 unmittelbar zugeordnet sind. Eine Batterie 205 mit nachgeschaltetem EOL-Indikator 206 versorgt die Baugruppen mit Energie. Nach Aktivierung über einen Reed-Schalter 207 wird über eine Empfängerspule 208, einen Empfänger 209, einen Programmverstärker 210 und eine Sicherheitsschaltung 211 zur Programmüberprüfung ein Programm- und Betriebsdatenregister 212 geladen, aus dem durch die Steuereinheit 202 Programmdaten abgefragt werden können. (Das Laden des Programmregisters erfolgt über ein Programmiergerät mit Telemetrieeinheit, wie es in Fig. 5 skizziert ist.)

Dem Schrittmacher 201 ist ein Herzkatheter K zugeordnet, das eine (rechts-)atrial sowie eine (rechts-)ventrikulär angeordnete Elektrodenanordnung EA bzw. EV mit konventionellen Sensing- und Stimulationsfunktionen umfaßt. Weiterhin ist ihm ein als solcher ebenfalls bekannter Aktivitätssensor Act zugeordnet, der die körperliche Aktivität des Patienten und damit dessen Belastung repräsentierende Signale abgibt.

Über die Elektrodenanordnungen EA und EV werden Herzaktionspotentiale erfaßt und über einen Eingangsverstärker 213 für atriale Signale und einen Eingangsverstärker 214 für ventrikuläre Signale (mit jeweils nachgeschalteter Störerkennungsschaltung 215 bzw. 216) der Steuereinheit 202 zugeführt. Die Meßsignale des Aktivitätssensors Act werden einer Auswertungseinheit 217 zugeführt. Das Ausgangssignal dieser Einheit 217 wird ebenfalls der Steuereinheit 202 für den Schrittmacherbetrieb zugeführt, wo es zur laufenden Einstellung der Stimulationsrate und/oder der AV-Verzögerung entsprechend dem dem nachgewiesenen Aktivitätsniveau entsprechenden metabolischen Bedarf des Patienten herangezogen wird, während über die intrakardial gemessenen Herzaktionspotentiale der Demand-Betrieb des Schrittmachers 201 gesteuert wird.

Wie oben unter Bezugnahme auf Fig. 4 und 5 beschrieben, wird die gültige Korrelation zwischen dem Parameterpaar Stimulationsrate/AV-Verzögerung und der Belastung bzw. Aktivität des Patienten während der periodischen Nachsorgeuntersuchungen eingestellt. Dazu umfaßt der Programm- und Betriebsparameterspeicher 212 einen durch Ausgangssignale der Auswertungseinheit 217 (d.h. durch aufbereitete Aktivitätssignale) frei adressierbaren Speicherbereich, in dem die gültigen f/AV-Paare für unterschiedliche Belastungszustände während der Untersuchung eingespeichert werden. Für den Normalbetrieb des Schrittmachers wird dann durch das aufbereitete Aktivitätssignal jeweils ein Speichereberich adressiert und das darin gespeicherte Parameterpaar zur Steuerung des Impulserzeugers abgerufen.

Die mit entsprechenden Parametern erzeugten Reizimpulse werden über je eine Ausgangsverstärkerschaltung 218 bzw. 219 an die Elektrodenanordnungen EA bzw. EV geliefert und von diesen an das reizbare Herzgewebe übertragen.

Die Blutsauerstoffsättigung kann natürlich auch zur Optimierung nur eines Schrittmacher-Betriebsparameters - etwa allein der AV-Verzögerung bei gegebener Stimulationsrate - genutzt werden, und die Programmierung des Schrittmachers kann durch weitere Messungen nach anderen Verfahren (etwa der körperlichen Aktivität direkt, durch Impedanzmessungen etc.) zusätzlich gestützt sein.

Figur 7 ist ein vereinfachtes Blockschaltbild einer Vorrichtung 1 zur extrakorporalen Bestimmung der Blutsauerstoffsättigung, in der Figur an einem Finger 2.

Die Vorrichtung umfaßt eine Sendestufe 1.1 mit einem senderseitigen Rot-Kanal 1.1a und einem senderseitigen Infrarot-Kanal 1.1b und eine Empfangsstufe 1.2 mit einem empfängerseitigen Rot-Kanal 1.2a und einem empfängerseitigen Infrarot-Kanal 1.2b. Die empfänger- und die senderseitigen Rot- bzw. Infrarot-Kanäle 1.1a und 1.2a bzw. 1.1b und 1.2b bilden zwei getrennte Meßkanäle 1a und 1b zur Ermittlung der Transmission bei sichtbarem Licht mit einer Wellenlänge von 660 bzw. bei Infrarotstrahlung mit einer Wellenlänge von 950 nm.

Die Sendestufe umfaßt eine bei 660 nm emittierende, d.h. rotleuchtende LED 3a mit einer Gleichspannungsversorgung 4a und einer Wechselspannungsversorgung 5a zur tonfrequenten (1kHz-)Modulation des ausgesandten Lichtes im ersten Kanal 1a. Sie umfaßt analog eine bei 950 nm infrarot-emittierende LED 3b mit einer Gleichspannungsversorgung 4b und einer Wechselspannungsversorgung 5b zur 1-kHz-Modulation der ausgesandten IR-Strahlung im zweiten Kanal 1b.

Die Strahlung beider LEDs wird in einen Abstandsbereich A eines (in der Figur nicht gezeigten) Gehäuses, in den der Finger 2 eingeführt ist, emittiert und von einem jeweils der LED 3a bzw. 3b gegenüberliegenden Fotodetektor (einer PIN-Diode) 6a bzw. 6b aufgenommen. Dem Fotodetektor 6a im Rot-Kanal 1a ist ein optisches Bandfilter 7a vorgeschaltet. Die vom jeweiligen Fotodetektor 6a bzw. 6b gelieferten Signale durchlaufen nachfolgend analoge Verarbeitungswege. Sie werden zunächst in einem Verstärker 8a bzw. 8b verstärkt, wonach sich der Signalweg verzweigt.

Ein erster Signalweg führt jeweils über ein 40-Hz-Tiefpaßfilter 9a bzw. 9b und ein 0,1Hz-Hochpaßfilter 10a bzw. 10b und liefert am Ausgang des letzteren ein Plethysmogramm-bzw. das eigentliche Meßsignal SO(R) bzw. SO(IR). Eine Quotientenbildung in einer nachgeordneten arithmetischen Verarbeitungsstufe 1.3 liefert daraus etwa einen Wert, aus dem anhand der in Fig. 3 gezeigten Kurve SaO2 ablesbar ist.

Ein zweiter Signalweg führt jeweils über ein 1-kHz-Bandpaßfilter 11a bzw. 11b, an dessen Ausgang kontinuierlich je ein erstes Korrektursignal S_{C1(R)} bzw. S_{C1(IR)} zur Ermittlung der Transmissionseffizienz der Probe (des Fingers 2) bereitsteht. Dies geschieht durch einen Vergleich der empfangenen Amplitude des 1-kHz-Pilotsignals mit dem ursprünglichen, aufmodulierten Pilotsignal in jeweils einer ersten Pilotsignal-Verarbeitungs- und Vergleichereinheit 1.4a bzw. 1.4b.

Ein dritter Signalweg führt jeweils über ein 3-kHz-Bandpaßfilter 12a bzw. 12b, an dessen Ausgang je ein zweites Korrektursignal S_{C2(R)} bzw. S_{C2(IR)} bereitsteht, das zum Nachweis harmonischer Verzerrungen infolge nichtlinearen Betriebs der Bauelemente genutzt werden kann. Dies geschieht wiederum durch vergleichende Verarbeitung mit dem ursprünglichen 1-kHz-Signal in jeweils einer zweiten Pilotsignal-Verarbeitungs- und Vergleichereinheit 1.5a bzw. 1.5b.

Die Verarbeitungsergebnisse der Stufen 1.3. 1.4a. 1.4b. 1.5a und 1.5b werden einer Anzeigeeinheit 1.6 des Oximeters zugeführt, wo sie für den Bediener dargestellt werden, der daraufhin ggf. über eine entsprechende (nicht gezeigte) Bedieneinheit bestimmte Einstellungen, insbesondere den Ansteuerstrom der LEDs, verändern kann.

Die Auswertung der Transmissionseffizienz ermöglicht es auch, bei Messungen an einem Patienten zu verschiedenen Zeiten sowie für Messungen an einer Patienten-Population normierte Daten zur Verfügung zu haben. Für einen Patienten können dazu die in beiden Kanälen gewonnenen Transmissions-Daten in einer Patienten-Datei gespeichert und frühere Werte mit den Werten aktueller Messungen verreichnet werden.

Treten harmonische Verzerrungen auf, die mittels des 3-kHz-Filters nachgewiesen werden können - was insbesondere bei sehr hoher Umgebungshelligkeit oder sehr "durchscheinender" Haut des Patienten infolge Übersteuerung der Fotodetektoren 6a oder 6b vorkommen kann, so kann auch unmittelbar durch S_{C2(R)} bzw. S_{C2(IR)} eine (in der Figur nicht gezeigte) Steuereinrichtung betätigt werden, die die Gleichstromversorgung 4a bzw. 4b derart ansteuert, daß der LED-Strom für die LED 3a bzw. 3b in vorgegebenen Stufen kalibriert absenkt, bis keine Verzerrung mehr auftritt, d.h. kein Signal S_{C2(R)} bzw. S_{C2(IR)} mehr nachweisbar ist. Auch diese Einstellung kann für einen speziellen Patienten in dessen Patientendatei gespeichert werden, so daß bei einer späteren Untersuchung gleich mit dieser Einstellung begonnen werden kann.

Wird sowohl im zweiten als auch im dritten Signalweg kein Signal empfangen, so ist die Meßsonde nicht angeschlossen oder das Sondenkabel defekt, und der Bediener erhält auf dem Display 1.6 eine entsprechende Information.

Ein genauere Darstellung der Schaltung und Bauelement-Bestückung der Sende- und der Empfangsstufe wird weiter unten unter Bezugnahme auf Fig. 8 bzw. die Figuren 10 und 11 gegeben.

Fig. 8 zeigt ein Anschlußschema sowie Bauelement-Spezifikationen für die (durch Einsatz je dreier LED im Rotund im Infrarot-Kanal leicht modifizierte) eigentliche Meßsonde gemäß Fig. 7. Die Bezugsziffern sind - bis auf die entsprechende Ersetzung der Ziffern 3a durch 3a' bzw. 3b durch 3b' - dieselben wie in Fig. 7, und die Schaltung wird als solche nicht nochmals beschrieben.

Die Anschlußbezeichnungen sind wie folgt zu verstehen:
- Rled :: Verbindung zur Rot-Stromquelle (4a, 5a in Fig. 4)
- Bright :: für den 3-LED-Betrieb +12V
- Dim :: für den 2-LED-Betrieb +12V, hat Priorität gegenüber Bright
- Irled :: Verbindung zur Infrarot-Stromquelle (4b, 5b in Fig. 4)
- ProbeID:: Reserve-Anschluß für eine spätere Kennzeichnung des Sondentyps
- Irout :: Ausgang des IR-Verstärkers 8b
- OV :: Masseanschluß für die Verstärker, separat mit zentraler Erde verbunden
- Rout :: Ausgang des Rot-Verstärkers 8a
- +12V :: positive Versorgungsleitung
- -12V :: negative Versorgungsleitung.

Die vier erstgenannten Signale können auf einer Doppelleitung mit einfacher Abschirmung übertragen werden, deren Abschirmung mit einem zentralen 0V-Bezugspunkt verbunden ist. Minimales Übersprechen wird durch 180°-Phasenverschiebung zwischen dem Rot- und dem Infrarot-Pilotton erreicht.

Figur 9a und 9b sind Darstellungen der Empfindlichkeitskurven des Rot- bzw. des IR-Empfangs-Bauelementes 6a (mit vorgeschaltetem optischem Filter 7a) bzw. 6b (mit integriertem optischen Filter) der in Fig. 7 gezeigten Vorrichtung.

Bei der in Fig. 8 spezifizierten Ausführung der Meßsonde wird als Detektor 6a im Rot-Kanal 1a eine PIN-Diode Siemens BPW34 verwendet, deren spektrale Empfindlichkeitskurve die Gestalt der gestrichelten Kurve in Fig. 9a hat. Es ist zu erkennen, daß der Detektor im Wellenlängenbereich von etwa 400 bis etwa 1100 nm empfindlich ist, während ein Nachweis nur um 660 nm benötigt wird und sowohl die Empfindlichkeit im kurz- als auch im langwelligeren Bereich die Meßgenauigkeit der Vorrichtung verschlechtert.

Der Detektor wird daher mit einem IR-Sperrfilter Edmund Scientific G39,424 sowie einem Filter zur Ausblendung kürzerer Wellenlängen versehen, wie es der Siemens BPW21-Fotodetektor aufweist. Insgesamt ergibt sich mit dem Komposit-Filter 7a dann die durchgezogene Transmissionskurve.

Fig. 9b zeigt die spektrale Empfindlichkeitskurve der als IR-Detektor verwendeten PIN-Diode Siemens BPW34F mit integriertem Tageslichtfilter. Es ist zu erkennen, daß unterhalb 750 nm praktisch keine Transmsission auftritt, so daß eine ausgezeichnete Kanaltrennung Rot-Infrarot und nahezu völlige Unempfindlichkeit gegenüber Störungen durch sichtbares Licht erreicht wird.

Figur 10 ist eine Detaildarstellung der LED-Ansteuerung bei einem gegenüber der Vorrichtung nach Fig. 7 modifiziertem Pulsoximeter 1', von dem in Fig. 10 nur die Sendestufe 1.1' gezeigt ist. Die Empfangsstufe kann gemäß Fig. 7 bzw. der nachfolgend erläuterten Fig. 11 ausgebildet sein.

Die Sendestufe umfaßt als Strahlungsemitter - wie bereits in Fig. 8 gezeigt - je eine aus drei LEDs bestehende Rot-Sendergruppe 3a' und Infrarot-Sendergruppe 3b'. Die Spezifikation der Sende-Bauelemente ist (wie auch für die anderen Bauelemente) in der Figur angegeben.

Der Rot- und der Infrarot-Kanal verfügen über getrennte spannungsgesteuerte Stromquellen. Ein Gleichstromsteuersignal (10 mA/V für Rot, 15 mA/V für Infrarot) wird jeweils über einen Widerstand 40a' bzw. 40b' und einen Knoten K1a bzw. K1b dem nicht-invertierenden Eingang eines Operationsverstärkers 41a' bzw. 41b' zugeleitet, dessen invertierender Eingang jeweils über einen Knoten K2a bzw. K2b einerseits mit dem Emitter eines npn-Transistors 42a' bzw. 42b' und andererseits über einen Widerstand 43a' bzw. 43b' mit Masse verbunden ist. Ein Wechselstromsteuersignal (für das 1-kHz-Pilotsignal) wird über einen Kondensator 44a' bzw. 44b' ebenfalls über den Knoten K1a bzw. K1b dem nicht-invertierenden Eingang des Operationsverstärkers 41a' bzw. 41b' zugeführt.

Die Ausgänge der Operationsverstärker 41a' bzw. 41b' sind jeweils mit der Basis der Transistoren 42a' bzw. 42b' verbunden. Die Kollektoren der Transistoren 42a' und 42b' sind entweder über die (vollständige) Sendeelementgruppe 3a' bzw. 3b' und eine Diode 45' mit einer +12V-Versorgungsspannung ("Bright") oder - im Falle der Benutzung nur je zweier LEDs - über jeweils zwei LEDs der Sendeelementgruppe und je eine Diode 46a' bzw. 46b' mit der +12V-Versorgungsspannung ("Dim") verbunden.

Die Operationsverstärker 41a', 41b' steuern in Abhängigkeit von den angelegten Gleich- und Wechselstrom-Steuersignalen in an sich bekannter Weise über die Transistoren 42a', 42b' in Basisschaltung deren Kollektorstrom und damit den Betriebsstrom der LED-Gruppen 3a' und 3b'.

Figur 11 ist eine Teil-Darstellung der Empfangsstufe, speziell der Auswertungsschaltung, eines gegenüber der Vorrichtung nach Fig. 7 etwas abgewandelten Pulsoximeters, wobei beide Kanäle gleichartig aufgebaut sind und hier nur der Rot-Kanal 1a' dargestellt ist. Mit Fig. 7 übereinstimmend ist die Führung des von der PIN-Diode 6a kommenden Meßsignals über drei getrennte Signalwege, die in Fig. 11 mit 1.21', 1.22' und 1.23' bezeichnet sind und auf denen Ausgangssignale S_{O(R)}, S_{C1(R)} bzw. S_{C2(R)} geliefert werden.

Im Signalweg 1.21' ist eine aus einem Widerstand 80a', einem weiteren Widerstand 81a', einem zu diesem parallel geschalteten Kondensator 82a', einem den Widerstand 81a' von Masse trennenden Kondensator 83a', einem Operationsverstärker 84a' und zwei weiteren Widerständen 85a' und 86a' gebildete Tiefpaßfilter- und Verstärkerschaltung vorgesehen, wobei der nicht-invertierende Eingang des Operationsverstärkers 84a' über die zueinander in Reihe geschalteten Widerstände 80a' und 81a' mit dem Ausgang des Fotodetektors und über den Kondensator 83a' mit Masse, sein invertierender Eingang über den Widerstand 85a' mit Masse und sein Ausgang über den Widerstand 86a' mit Masse, über den Kondensator 82a' mit dem Verbindungspunkt zwischen den Reihen-Widerständen 80a' und 81a' sowie mit der nächsten Verarbeitungsstufe verbunden ist.

Die Darstellung in der rechten unteren Ecke von Fig. 11 zeigt die Verbindung des Operationsverstärkesr (sowie auch der weiteren, weiter unten beschriebenen Operationsverstärker) mit der Stromversorgung und - über zwei nicht mit Bezugsziffern versehene Kondensatoren - mit Masse.

Vom Ausgang des Operationsverstärkers 84a' gelangt das Signal in eine aus einem Kondensator 100a', einem weiteren Kondensator 101a', einer zu diesem parallel liegenden Reihenschaltung aus zwei Widerständen 102a', 103a', einer den Kondensator 101a' von Masse trennenden Serienschaltung aus zwei Widerständen 104a', 105a', einem Operationsverstärker 106a' und zwei weiteren Widerständen 107a' und 108a' gebildete Hochpaßfilter- und Verstärkerschaltung, bei der der nicht-invertierende Eingang des Operationsverstärkers 106a' über die zueinander in Reihe geschalteten Kondensatoren mit dem Eingang dieser Stufe und über die Widerstände 104a', 105a' mit Masse, sein invertierender Eingang über den Widerstand 107a' mit Masse und sein Ausgang über den Widerstand 108a' mit Masse und über die Serien-Widerstände 102a', 103a' mit dem Verbindungspunkt zwischen den Kondensatoren 100a', 101a' verbunden ist und gleichzeitig den Ausgang des ersten Signalweges 1.21' bildet.

Die gezeigte Operationsverstärkerschaltung realisiert - neben dem 40Hz-Tiefpaß und dem 0,1Hz-Hochpaß als Filter mit näherungsweiser Bessel-Charakteristik und niedrigen Phasen-Verzerrungen - einen NIC-Konverter ("Transimpedance Amplifier"), mit dem die Nichtlinearität der optischen Bauelemente in gewisser Weise kompensiert wird.

Der zweite und dritte Signalweg 1.22' und 1.23' sind gleichartig aufgebaut und unterscheiden sich nur in der (in der Figur angegebenen) Dimensionierung der Baulemente, so daß in der Figur nur der zweite Signalweg mit Bezugsziffern versehen ist und hier nur dieser beschrieben wird.

Er enthält einen Widerstand 110a', eine zu diesem in Reihe geschaltete Parallelschaltung aus zwei Kondensatoren 111a', 112a' und einen Operationsverstärker 113a', dessen invertierender Eingang über die vorgenannten Elemente einerseits mit dem Eingang der Stufe und andererseits mit einer RC-Parallelschaltung 114a' verbunden ist und dessen nichtinvertierender Eingang über den Abgriff eines Potentiometers 115a' mit Masse verbunden ist.

Der Ausgang des Operationsverstärkers 113a' liegt einerseits über einen Widerstand 116a' und das Potentiometer 115a' an Masse und ist andererseits zum einen mit der RC-Parallelschaltung 114a' und zum anderen mit dem Eingang einer in Durchlaßrichtung geschalteten Diode 117a' verbunden. Deren Ausgang bildet - von Masse durch eine RC-Parallelschaltung aus einem Elektrolytkondensator 118a' und einem Widerstand 119a' getrennt - den Ausgang des Signalweges 1.22'.

Die Funktion des zweiten und dritten Signalweges wurde bereits oben unter Bezugnahme auf Fig. 7 erläutert. Mit der gezeigten Schaltung werden mit nur einem Operationsverstärker pro Stufe Filter hoher Güte realisiert.

Abweichungen von den gezeigten Schaltungen sind dem Fachmann jederzeit möglich, wobei auch das Prinzip des Nachweises harmonischer Verzerrungen über ein Pilotsignal modifizierbar ist.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

## Patentansprüche

1. Anordnung zur Festlegung eines ersten Betriebsparameters eines implantierten, extern programmierbaren Herzstimulators (PM; 201), insbesondere der Stimulationsrate oder der AV-Verzögerungszeit, aufgrund von am Patienten gemessenen Werten einer seinen metabolischen Zustand reflektierenden, nicht-elektrischen Größe, mit einem über eine Telemetrieinrichtung (Tel) mit dem Herzstimulator (PM; 201) verbindbaren externen Programmiergerät (Pr), einer Sensoranordnung (3a, 3b, 6a, 6b; 3a', 3b') zur Erfassung der Größe und einer Steuer- und Auswertungseinrichtung (1.1, 1.2; 1.1') zur Steuerung des Betriebs der Sensoranordnung und zur Bestimmung des Wertes der nicht-elektrischen Größe, wobei ein Eingang (Pr/I) des Programmiergerätes (Pr) mit dem Ausgang (1/O) der Steuer- und Auswertungseinrichtung (1.1, 1.2; 1.1') verbunden ist und deren Ausgangssignal empfängt und das Programmiergerät Mittel (Pr/MAux, Pr/Com, Pr/Cri, Pr/Ti) zur selbsttätigen Durchführung und Auswertung mindestens einer Meßreihe für die nicht-elektrische Größe in Abhängigkeit von verschiedenen, über die Telemetrieeinrichtung am Herzstimulator eingestellten Werten des Betriebsparameters und Mittel zur Festlegung eines in Relation zum ersten Betriebsparameter optimierten Betriebsparameters entsprechend dem Auswertungsergebnis der Meßreihe oder Meßreihen aufweist,
**dadurch gekennzeichnet, daß** dem Herzstimulator (PM) eine weitere Sensoranordnung (Act) zur kontinuierlichen oder quasi-kontinuierlichen Erfassung einer die körperliche Aktivität oder Belastung des Patienten reflektierenden weiteren Größe während seines normalen Betriebs zugeordnet ist und der Herzstimulator (PM) eine mit dem Ausgang der weiteren Sensoranordnung verbundene, insbesondere als Matrixspeicher ausgeführte, interne Speichereinheit (212) zur Speicherung des jeweils eingestellten Wertes des Betriebsparameters (f, AV) in Abhängigkeit vom aktuellen Meßwert der weiteren Größe aufweist, dessen Dateneingang mit dem Ausgang des Programmiergerätes (Pr) verbindbar ist derart, daß im Rahmen eines Einstellvorganges jeweils in Zuordnung zu einem aktuellen Meßwert der weiteren Größe ein vom Programmiergerät gelieferter Wert des Betriebsparameters gespeichert wird.

2. Anordnung nach Anspruch 1, **dadurch gekennzeichnet, daß** das Programmiergerät (Pr) einen, insbesondere als Matrixspeicher ausgebildeten, Hilfsspeicher (Pr/MAux) zur Speicherung von Meßwerten der nicht-elektrischen Größe in Zuordnung zu Betriebsparameterwerten, nach deren Einstellung sie aufgenommen wurden, aufweist.

3. Anordnung nach Anspruch 1 oder 2, **dadurch gekennzeichnet, daß** das Programmiergerät (Pr) eine mit dem Datenausgang des Hilfsspeichers (Pr/MAux) verbundene Vergleichereinheit (Pr/Com) zur Auswertung der Meßwerte der nicht-elektrischen Größe, einen mit einem Steuereingang der Vergleichereinheit verbundenen Kriterien-Festwertspeicher (Pr/MCri) zur Speicherung mindestens eines Vergleichskriteriums für diese Auswertung und ein eingangsseitig mit einem Adreßausgang des Hilfsspeichers Pr/MAux sowie mit dem Ausgang der Vergleichereinheit (Pr/Com) und ausgangsseitig mit der Telemetrieeinheit (Tel) verbundenes Betriebsparameter-Register (Pr/Reg) zur Zwischenspeicherung der zu den aktuell in der Vergleichereinheit ausgewerteten Meßwerten gehörigen Werte des Betriebsparameters sowie des im Ergebnis der Auswertung als optimal bestimmten Wertes des Betriebsparameters aufweist.

4. Anordnung nach einem der vorangehenden Ansprüche, **dadurch gekennzeichnet, daß** das Programmiergerät (Pr) eine Zeitablaufsteuerung (Pr/Ti) zur zeitlichen Steuerung des Ablaufs einer Meßreihenserie derart, daß in einem vorbestimmten Zeittakt in vorbestimmter Folge verschiedene Wertepaare des ersten und zweiten Betriebsparameters am Schrittmacher (PM) eingestellt und zusammen mit dem jeweils erhaltenen Meßwert der nicht-elektrischen Größe ausgegeben bzw. gespeichert werden, aufweist.

5. Anordnung nach einem der vorangehenden Ansprüche, **dadurch gekennzeichnet, daß** der erste Betriebsparameter die Stimulationsrate (f), der zweite Betriebsparameter die AV-Verzögerungszeit (AV) und die nicht-elektrische Größe die Blutsauerstoffsättigung (SaO₂) ist.

6. Anordnung nach Anspruch 5, **dadurch gekennzeichnet, daß** die Sensoranordnung mindestens je ein sichtbares Licht sowie Infrarotstrahlung aussendendes Bauelement (3a, 3b; 3a', 3b') sowie ein das sichtbare Licht und die Infrarotstrahlung nach Durchgang durch einen durchbluteten Körperbereich (2; H) empfangendes Bauelement (6a, 6b) umfaßt.

7. Anordnung nach Anspruch 6, **dadurch gekennzeichnet, daß** je ein das sichtbare Licht und die Infrarotstrahlung empfangendes Bauelement (6a, 6b) vorgesehen ist und daß das das sichtbare Licht aussendende und das das sichtbare Licht empfangende Bauelement (3a, 6a; 3a', 6a) einen ersten Meßkanal (1a) sowie das die Infrarotstrahlung aussendende und das die Infrarotstrahlung empfangende Bauelement (3b, 6b; 3b', 6b) einen zweiten, vom ersten getrennten, Meßkanal (1b) bilden und die Steuer-und Auswertungseinrichtung (1.1, 1.2; 1.1') so ausgebildet ist, daß der erste und der zweite Meßkanal (1a, 1b) gleichzeitig betrieben und die in beiden Meßkanälen erhaltenen Meßsignale im wesentlichen gleichzeitig verarbeitet werden.

8. Anordnung nach einem der Ansprüche 5 bis 7, **dadurch gekennzeichnet, daß** die Steuer- und Auswertungseinrichtung (1.1, 1.2; 1.1') Mittel (5a, 5b) zum Erzeugen und Anlegen einer Wechselspannung an die aussendenden Bauelemente (3a, 3b; 3a', 3b') zur Modulation der emittierten Strahlung sowie mindestens eine Verarbeitungsstufe (11a, 12a, 11b, 12b) zur Auswertung des modulierten Anteils der empfangenen Strahlung aufweist.

9. Anordnung nach Anspruch 8, **dadurch gekennzeichnet, daß** eine Verarbeitungsstufe (12a, 1.5a, 12b, 1.5b) zur Bestimmung harmonischer Verzerrungen und/oder eine Verarbeitungsstufe (11a, 1.4a, 11b. 1.4b) zur Bestimmung des Transmissionsgrades der empfangenen Strahlung vorgesehen ist.

10. Anordnung nach Anspruch 9, **dadurch gekennzeichnet, daß** eine Anzeigeeinheit (1.6) für den Transmissionsgrad und/oder harmonische Verzerrungen und eine Vorrichtung zur, vorzugsweise selbsttätigen, Justierung des Betriebsstromes der aussendenden Bauelemente (3a, 3a', 3b, 3b') vorgesehen ist.

11. Anordnung nach Anspruch 10, **dadurch gekennzeichnet, daß** eine Speichereinheit (1/M) zur patientenbezogenen Speicherung des ermittelten Transmissionsgrades und/oder der Justierstellung des Betriebsstromes vorgesehen ist.

## Claims

1. Arrangement for fixing a first operating parameter of an implanted, externally programmable pacemaker (PM; 201), in particular the stimulation rate or the AV delay time, on the basis of values measured on the patient of a non-electrical quantity reflecting his metabolic state, comprising an external programming device (Pr) which can be connected to the pacemaker (PM; 201) via a telemetric device (Tel), a sensor arrangement (3a, 3b, 6a, 6b; 3a', 3b') for detecting the quantity and a control and evaluation device (1.1, 1.2; 1.1') for controlling the operation of the sensor arrangement and for determining the value of the non-electrical quantity, wherein an input (Pr/I) of the programming device (Pr) is connected to the output (l/O) of the control and evaluating device (1.1, 1.2; 1.1')and receives its output signal and the programming device comprises means (Pr/MAux, PR/Com, Pr/Cri, Pr/Ti) for automatically carrying out and evaluating at least one series of measurements for the non-electrical quantity as a function of various values of the operating parameter adjusted on the pacemaker via the telemetric device, and means for fixing an operating parameter optimised in relation to the first operating parameter according to the evaluation result of the one or more series of measurements, **characterised in that** a further sensor arrangement (Act) for continuously or virtually continuously detecting a further quantity reflecting the physical activity or stress of the patient during his normal business is associated with the pacemaker (PM) and the pacemaker (PM) comprises an internal memory unit (212) connected to the output of the further sensor arrangement and designed, in particular, as a matrix memory, for storing the respectively adjusted value of the operating parameter (f,AV) as a function of the current measured value of the further quantity, of which the data input can be connected to the output of the programming device (Pr) such that, in the framework of an adjustment process, a value of the operating parameter supplied by the programming device is stored in each case in allocation to a current measured value of the further quantity.

2. Arrangement according to claim 1, **characterised in that** the programming device (Pr) comprises an auxiliary memory (Pr/MAux) designed, in particular, as a matrix memory, for storing measured values of the non-electrical quantity in allocation to the operating parameter values, according to the adjustment of which they were recorded.

3. Arrangement according to claim 1 or 2, **characterised in that** the programming device (Pr) comprises a comparator (Pr/Com) connected to the data output of the auxiliary memory (Pr/MAux) for evaluating the measured values of the non-electrical quantity, a criteria read-only memory (Pr/MCri) connected to a control input of the comparator for storing at least one comparison criterion for this evaluation and an operating parameter register (Pr/Reg) connected at the input side to an address output of the auxiliary memory Pr/MAux and to the output of the comparator (Pr/Com) and at the output side to the telemetric unit (Tel) for buffering the values of the operating parameter belonging to the current measured values evaluated in the comparator and the value of the operating parameter deemed optimal as a result of the evaluation.

4. Arrangement according to any of the preceding claims, **characterised in that** the programming device (Pr) comprises a time lapse controller (Pr/Ti) for temporally controlling the course of a series of measurements such that, in a predetermined clock, various pairs of values of the first and second operating parameter are adjusted in a predetermined sequence on the pacemaker (PM) and together with the respectively obtained measured value of the non-electrical quantity are output or stored.

5. Arrangement according to any of the preceding claims, **characterised in that** the first operating parameter is the stimulation rate (f), the second operating parameter the AV delay time (AV) and the non-electrical quantity is the blood oxygen saturation (SaO₂).

6. Arrangement according to claim 5, **characterised in that** the sensor arrangement comprises at least one respective component (3a, 3b; 3a', 3b') emitting a visible light and infrared radiation and a component (6a, 6b) receiving the visible light and the infrared radiation after passage through a body region (2; H) supplied with blood.

7. Arrangement according to claim 6, **characterised in that** a respective component (6a, 6b) receiving the visible light and the infrared radiation is provided and **in that** the component (3a, 6a; 3a', 6a) emitting the visible light and receiving the visible light from a first measuring duct (1a) and the component (3b, 6b; 3b', 6b) emitting the infrared radiation and receiving the infrared radiation from a second measuring duct (1b), separate from the first, and the control and evaluating device (1.1, 1.2; 1.1') is designed in such a way that the first and the second measuring ducts (1a, 1b) are operated simultaneously and the measured signals obtained in the two measuring ducts are processed substantially simultaneously.

8. Arrangement according to any claims 5 to 7, **characterised in that** the control and evaluating device (1.1, 1.2; 1.1') comprises means (5a, 5b) for generating and applying an alternating voltage to the emitting components (3a, 3b; 3a', 3b') for modulating the emitted radiation and at least one processing stage (11a, 12a, 11b, 12b) for evaluating the modulated portion of the received radiation.

9. Arrangement according to claim 8, **characterised in that** a processing stage (12a, 1.5a, 12b, 1.5b) is provided for determining harmonic distortions and/or a processing stage (11a, 1.4a, 11b, 1.4b) is provided for determining the degree of transmission of the received radiation.

10. Arrangement according to claim 9, **characterised in that** a display unit (1.6) is provided for the degree of transmission and/or harmonic distortions and a device is provided for, preferably automatically, adjusting the operating current of the emitting components (3a, 3a', 3b, 3b').

11. Arrangement according to any claim 10, **characterised in that** a memory unit (1/M) is provided for storing the degree of transmission ascertained and/or for adjusting the operating current in relation to the patient.

## Revendications

1. Dispositif pour déterminer un premier paramètre de fonctionnement d'un stimulateur cardiaque implantable (PM;201) programmable de l'extérieur, notamment de la cadence de stimulation ou de la durée de retardement AV sur la base de valeurs, mesurées chez le patient, d'une grandeur non électrique, qui reproduit son état métabolique, comportant un appareil externe de programmation (Pr) , qui peut être relié par l'intermédiaire d'un dispositif de télémétrie (Tel) au stimulateur cardiaque (PM; 201), un dispositif de capteurs (3a,3b,6a,6b;3a',3b') pour détecter la grandeur et le dispositif de commande et d'évaluation (1.1, 1.2; 1.1') pour commander le fonctionnement du dispositif de capteurs et pour déterminer la valeur de la grandeur non électrique, dans lequel une entrée (Pr/I) de l'appareil de programmation (Pr) est reliée à la sortie (1/O) du dispositif de commande et d'évaluation (1.1, 1.2; 1.1') et reçoit le signal de sortie de ce dispositif et l'appareil de programmation comporte des moyens (Pr/MAux, Pr/Com, Pr/Cri, Pr/Ti) pour l'exécution et l'évaluation automatiques d'au moins une série de mesures pour la grandeur non électrique en fonction de différentes valeurs du paramètre de fonctionnement, réglées dans le stimulateur cardiaque au moyen du dispositif de télémétrie, et des moyens pour déterminer un paramètre de fonctionnement optimisé par rapport au premier paramètre de fonctionnement, conformément au résultat de l'évaluation de la ou des séries de mesure,
**caractérisé en ce qu'**au stimulateur cardiaque (PM) est associé un autre dispositif de capteurs (Act) servant à détecter d'une manière continue ou quasi continue une autre grandeur, qui reproduit une activité corporelle ou une sollicitation corporelle du patient, pendant son fonctionnement normal, et que le stimulateur cardiaque (PM) comporte une unité de mémoire interne (212) qui est reliée à la sortie de l'autre dispositif de capteurs, notamment réalisée en tant que mémoire en forme de matrice, pour la mémorisation de la valeur respectivement réglée du paramètre de fonctionnement (f, AV) en fonction de la valeur de mesure réelle de l'autre grandeur, que son entrée de données peut être reliée à la sortie de l'appareil de programmation (Pr) de telle sorte que dans le cadre d'un processus de réglage respectivement en association avec une valeur de mesure actuelle de l'autre grandeur, une valeur délivrée par l'appareil de programmation, du paramètre de fonctionnement est mémorisée respectivement en association à une autre valeur de mesure actuelle de l'autre grandeur.

2. Dispositif selon la revendication 1, **caractérisé en ce que** l'appareil de programmation (Pr) possède une mémoire auxiliaire (Pr/MAux), agencée notamment sous la forme d'une mémoire en forme de matrice, pour la mémorisation de valeurs de mesure de la grandeur non électrique en association avec des valeurs des paramètres de fonctionnement, en fonction du réglage desquelles elles ont été enregistrées.

3. Dispositif selon la revendication 1 ou 2, **caractérisé en ce que** l'appareil de programmation (Pr) comporte une unité formant comparateur (Pr/Com), reliée à la sortie de données de la mémoire auxiliaire (Pr/Maux) et servant à évaluer les valeurs de mesure de la grandeur non électrique, une mémoire morte (Pr/Cri) pour un critère, reliée à l'entrée de commande de l'unité formant comparateur et servant à mémoriser au moins un critère de comparaison pour cette évaluation et un registre (Pr/Reg) de paramètre de fonctionnement relié à la sortie de l'unité formant comparateur (Pr/Com) et, côté sortie, à l'unité de télémétrie (Tel), pour la mémorisation temporaire des valeurs du paramètre de fonctionnement associées aux valeurs de mesure évaluées actuellement dans l'unité formant comparateur, ainsi que de la valeur du paramètre de fonctionnement, déterminée comme optimale en tant que résultat de l'évaluation.

4. Dispositif selon l'une des revendications précédentes, **caractérisé en ce que** l'appareil de programmation (Pr) possède une unité (Pr/Ti) d'exécution temporelle du déroulement d'une série de mesures de telle sorte qu'à une cadence prédéterminée, différents couples de valeurs des premier et second paramètres de fonctionnement sont réglés dans le stimulateur (PM) et sont délivrés ou mémorisés conjointement avec la valeur de mesure respectivement obtenue de la grandeur non électrique.

5. Montage selon l'une des revendications précédentes, **caractérisé en ce que** le premier paramètre de fonctionnement est la cadence de stimulation (f), le second paramètre de fonctionnement est le temps de retard AV (AV) et la grandeur non électrique est la saturation du champ en oxygène (SaO₂).

6. Dispositif selon la revendication 5, **caractérisé en ce que** le dispositif de capteurs comporte au moins respectivement un composant (3a,3b;3a',3b') qui émet une lumière visible ainsi qu'un rayonnement infrarouge, ainsi qu'un composant (6a,6b) qui reçoit la lumière visible et reçoit le rayonnement infrarouge après traversée d'une zone du corps (2;H) parcourue par le sang.

7. Dispositif selon la revendication 6, **caractérisé en ce qu'**il est prévu respectivement un composant (6a, 6b), qui reçoit la lumière visible et le rayonnement infrarouge, et le composant (3a,6a;3a',6), qui émet la lumière visible et le composant (3a,6a;3a',6) reçoit la lumière visible, forment premier un canal de mesure (1a) et le composant (3b,6b;3b',6b), qui émet le rayonnement infrarouge, et le composant (3b,6b,3b',6B), qui reçoit le rayonnement infrarouge, forment un second canal de mesure (1b) séparé du premier et que le dispositif de commande et d'évaluation (1.1, 1.2; 1.1') est agencé de telle sorte que les premier et second canaux de mesure (1a,1b) fonctionnent simultanément et que les signaux de mesure obtenus avec les deux canaux de mesure sont traités essentiellement simultanément.

8. Dispositif selon l'une des revendications 5 à 7, **caractérisé en ce que** le dispositif de commande et d'évaluation (1.1, 1.2; 1.1') comporte des moyens (5a,5b) pour produire et appliquer une tension alternative aux composants émetteurs (3a,3b;3a',3b') pour moduler le rayonnement émis, ainsi qu'au moins un étage de traitement (11a,12a, 11b,12b) pour l'évaluation de la composante modulée du rayonnement reçu.

9. Dispositif selon la revendication 8, **caractérisé en ce qu'**un étage de traitement (12a, 1.5a, 12b, 1.5b) est prévu pour déterminer des distorsions harmoniques et/ou un étage de traitement (11a, 1.4a, 11b, 1.4b) est prévu pour déterminer le degré de transmission du rayonnement reçu.

10. Dispositif selon la revendication 9, **caractérisé en ce qu'**il est prévu une unité d'affichage (1.6) pour le degré de transmission et/ou les distorsions harmoniques et un dispositif pour ajuster, de préférence automatiquement, le courant de fonctionnement des composants émetteurs (3a,3a',3b,3b').

11. Dispositif selon la revendication 10, **caractérisé en ce qu'**il est prévu une unité de mémoire (1/M) pour la mémorisation, rapportée au patient, du degré de transmission déterminé et/ou de la position d'ajustement du courant de fonctionnement.
